# EUROPEAN PATENT APPLICATION

(11) **EP 2 522 277 A1**
(43) Date of publication of application: **14.11.2012**
(21) Application number: 10818874.9
(22) Date of filing: 24.09.2010
(51) Int. Cl.: A61B 6/14, A61B 6/03

(54) **TWO-DIMENSIONAL IMAGE DISPLAY DEVICE**

(30) Priority: 24.09.2009 JP 2009219797
(71) Applicant: iCAT Corporation, Osaka-Shi, Osaka 532-0011 (JP)
(72) Inventor: SOGO, Motofumi, Osaka-Shi Osaka 532-0011 (JP); SAIGAN, Masaya, Osaka-Shi Osaka 532-0011 (JP)
(74) Representative: Plougmann & Vingtoft A/S
(86) International application number: PCT/JP2010/066588
(87) International publication number: WO 2011/037202

(57) **Abstract**

The purpose of the present invention is to provide a two-dimensional image display device that generates and displays a tomographic image on a panorama-orthogonal cross-sectional plane, which extends in superior-inferior direction and is orthogonal to a panoramic cross-sectional plane, from three-dimensional image information generated from X-ray CT photographic data of a head and neck region. The panorama-orthogonal cross-sectional plane is shiftable in a mesial-distal direction along the panoramic cross-sectional plane. Furthermore, the panorama-orthogonal cross-sectional plane is tiltable around the line of intersection formed by the panorama-orthogonal cross-sectional plane and a horizontal cross-sectional plane. The image display device displays a tomographic image of the head and neck region that is on the shifted and tilted panorama-orthogonal cross-sectional plane.

## Description

### TECHNICAL FIELD

The present invention relates to a two-dimensional image display device that generates and displays the cross-sectional images of a head and neck region from three-dimensional X-ray CT photographic data. The image display device includes an image display means (image display viewer), which displays the three-dimensional image of a jaw bone as well as a horizontal plane, a panoramic plane and a panorama-orthogonal plane. The panorama-orthogonal plane is tilted, rotated, shifted and slid. The image display viewer generates and displays the cross-sectional images on the panorama-orthogonal plane as well as the horizontal plane and panoramic plane.

### BACKGROUND OF THE INVENTION

In the field of medicine, X-ray CT imaging (hereafter, also referred as 'CT imaging') has been traditionally used to obtain a tomographic image inside the patient. In recent years, CT imaging has also been available in the dental field to obtain a detailed tomographic image of the maxillofacial region.

Although the images captured by CT imaging are tomographic images, namely two-dimensional analog information of the photographed subject, the tomographic images are convertible to three-dimensional digital information, and the three-dimensional image of the photographed subject can be displayed on a display. Therefore, dentists can obtain a maxillofacial tomographic image, viewing the three-dimensional maxillofacial image of the patient on the display

However, it was difficult to obtain a tomographic image of a desired maxillofacial cross-section even though the dentist could see the three-dimensional image on the display This was because the tomographic images that could be obtained by the conventional CT imaging technique were limited to a body-axial cross-section, a coronal cross-section and a sagittal cross-section.

In recent years, an imaging technique called curved MPR imaging has been developed. This is a technique to generate and display new tomographic images by processing already-captured images. This technique enables to obtain an image close to the dental orthopantomography. Such a tomographic image is called a panoramic cross-sectional image, and it enables more effective jawbone diagnosis. Furthermore, the curved MPR imaging technique also enables to obtain an image of the cross-section orthogonal to the panoramic cross-section (also called 'panorama-orthogonal cross-section'). This imaging technique enabled the dentist to obtain an image close to the shape that the dentist imagines based on what he sees during the treatment.

However, there still remains a problem in that the panorama-orthogonal cross-section can display inside of the jaw bone only vertically from the three-dimensional image.

Patent Document 1: International Publication WO2006/033483

### SUMMARY OF THE INVENTION

### Problem to be Solved by the Invention

The present invention was developed based on such a problem. The purpose of the present invention is to provide a device that generates and displays a desired two-dimensional image from the captured photographic data of a human body.

### Means to Solve the Problem

The present invention provides a two-dimensional image display device for detecting body information in the jaw bone by obtaining image information on a panorama-orthogonal cross-section, which extends in a superior-inferior direction and is orthogonal to a panoramic cross-section, from the three-dimensional image information generated from X-ray CT photographic information of a head and neck region.

The two-dimensional image display device includes at least a three-dimensional image display means, a horizontal cross-sectional image display means, a panoramic cross-sectional image display means, and a panorama-orthogonal cross-sectional image display means. The three-dimensional image display means displays a three-dimensional image of the head and neck region. The horizontal cross-sectional image display means displays a two-dimensional cross-sectional image on a horizontal plane defined in the three-dimensional image display means. The panoramic cross-sectional image display means displays a two-dimensional cross-sectional image on a panoramic cross-sectional plane. And, the panorama-orthogonal cross-sectional display means displays a two-dimensional image on a panorama-orthogonal cross-sectional plane.

The panorama-orthogonal cross-sectional plane is shiftable in a mesial-distal direction along the panoramic cross-section. Furthermore, the panorama-orthogonal cross-sectional plane is tiltable toward the mesial-distal direction around the rotational axis, which is the line of intersection formed by the panorama-orthogonal cross-sectional plane and the horizontal cross-sectional plane. The panorama-orthogonal cross-section display means displays a two-dimensional image in the jaw bone as body information that is on the shifted and tilted panorama-orthogonal cross-sectional plane.

According to another aspect of the two-dimensional image display device, the panorama-orthogonal cross-sectional plane is newly set and moved along the panoramic cross-sectional plane. The two-dimensional image on the panorama-orthogonal cross-sectional plane is displayed on the display Furthermore, the panorama-orthogonal cross-sectional plane can be tilted.

A tilted panorama-orthogonal cross-sectional plane is slid in the direction of tilt angle. The panorama-orthogonal cross-section display means displays the two-dimensional image inside the jaw bone located on the tilted and slid panorama-orthogonal cross-sectional plane as body information.

In other words, when the panorama-orthogonal cross-sectional plane is tilted around the line of intersection formed by the panorama-orthogonal cross-sectional plane and the horizontal cross-sectional plane, the panorama-orthogonal cross-sectional plane can be slid in the tilting direction, maintaining the tilt angle.

The tilted panorama-orthogonal cross-sectional plane is shiftable in the mesial-distal direction along the panoramic cross-section. The panorama-orthogonal cross-section display means displays a two-dimensional image in the jaw bone as body information that is on the shifted and tilted panorama-orthogonal cross-sectional plane.

Furthermore, after the panorama-orthogonal cross-sectional plane is tilted around the line of intersection formed by the panorama-orthogonal cross-sectional plane and the horizontal cross-sectional plane, the panorama-orthogonal cross-sectional plane can be shifted along the panoramic cross-sectional plane, maintaining the tilting orientation.

The panorama-orthogonal cross-sectional plane is rotatable around the line of intersection formed by the panorama-orthogonal cross-sectional plane and the panoramic cross-sectional plane. The panorama-orthogonal cross-section display means displays the two-dimensional image in the jaw bone as body information that is on the rotated panorama-orthogonal cross-sectional plane.

In other words, the panorama-orthogonal cross-sectional plane can be rotated around the rotational axis that is the line of intersection formed by the panorama-orthogonal cross-sectional plane and the panoramic cross-sectional plane.

The rotated panorama-orthogonal cross-sectional plane is slidable in the direction of the rotational angle of the panorama-orthogonal cross-sectional plane. The panorama-orthogonal cross-section display means displays the two-dimensional image in the jaw bone as body information that is on the rotated and slid panorama-orthogonal cross-sectional plane.

In other words, when the panorama-orthogonal cross-sectional plane is rotated around the line of intersection formed by the panorama-orthogonal cross-sectional plane and the panoramic cross-sectional plane, the panorama-orthogonal cross-sectional plane can be slid in the extended direction of the rotated panorama-orthogonal cross-sectional plane, maintaining the rotated orientation.

The rotated panorama-orthogonal cross-section is shiftable in the mesial-distal direction along the panoramic cross-section. The panorama-orthogonal cross-section display means displays the two-dimensional image in the jaw bone as body information that is on the shifted panorama-orthogonal cross-sectional plane.

In other words, after the panorama-orthogonal cross-sectional plane is rotated, the panorama-orthogonal cross-sectional plane can shift along the panoramic cross-sectional plane, maintaining such orientation.

### BRIEF DESCRIPTION OF THE DRAWINGS

Fig. 1 is a display image of the image display device in which the panorama-orthogonal cross-sectional plane is at a default state.
Fig. 2 is a display image of the image display device in which the panoramic cross-sectional plane is being configured.
Fig. 3 is a display image of the image display device in which the panorama-orthogonal cross-sectional plane has been shifted.
Fig. 4 is a display image of the image display device in which the panorama-orthogonal cross-sectional plane has been further shifted.
Fig. 5 is a display image of the image display device in which the panorama-orthogonal cross-sectional plane has been tilted.
Fig. 6 is a display image of the image display device in which the panorama-orthogonal cross-sectional plane has been further tilted.

### DETAILED DESCRIPTION OF THE INVENTION

The image display device converts a collection of two-dimensional analog images, which are the tomographic images of a patient's head and neck region obtained from X-ray CT photographic data, into DICOM data, which is three-dimensional digital information. The three-dimensional image is displayed on the display based on the DICOM data. If the dentist specifies a desired point or plane in the three-dimensional image, he can view the image of this corresponding cross-section.

Generally, the three-dimensional image of the head and neck region of the patient is configured by position information and X-ray information by each region unit (voxcel). The three-dimensional image is displayed as a collection of voxcels in the head and neck region.

In more detail, a three-dimensional image display means of the image display device retrieves the position information and X-ray information the DICOM data contain. Then, the three-dimensional image display means superimposes the position information and the X-ray information in the space in the window on the display. More specifically, the three-dimensional image display means displays the X-ray information in the corresponding voxcel. By repeating this for all the voxcels in the space, the three-dimensional image of head and neck region is constructed and displayed in the window.

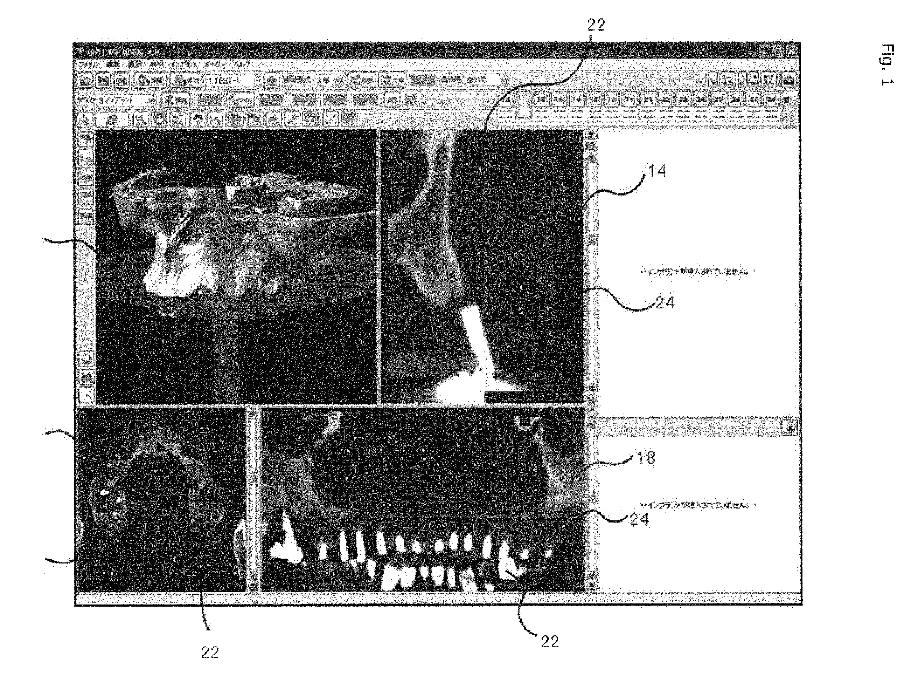

FIG. 1 shows an example of the display image of head and neck region of an actual patient. The upper left window 12 displays the three-dimensional image of the patient's head and neck region, particularly the maxilla.

Seeing the three-dimensional image, the dentist can specify coordinate planes in the window 12. Then, the cross-sectional images of the head and neck region corresponding to the coordinate planes are displayed in other windows. Such cross-sections may include a cross-section perpendicular to the body axis of the head and neck region, a cross-section extending in the anterior-posterior direction of the head and neck region, a cross-section extending in the medial-lateral direction of the head and neck region, and a cross-section curved along the dental arch (so-called panoramic image).

In the case of FIG. 1, the three-dimensional window 12 is displayed in the upper left of the screen, a panorama-orthogonal cross-section window 14 in the upper right, a horizontal cross-section window 16 in the lower left and a panoramic cross-section window 18 in the lower right, respectively

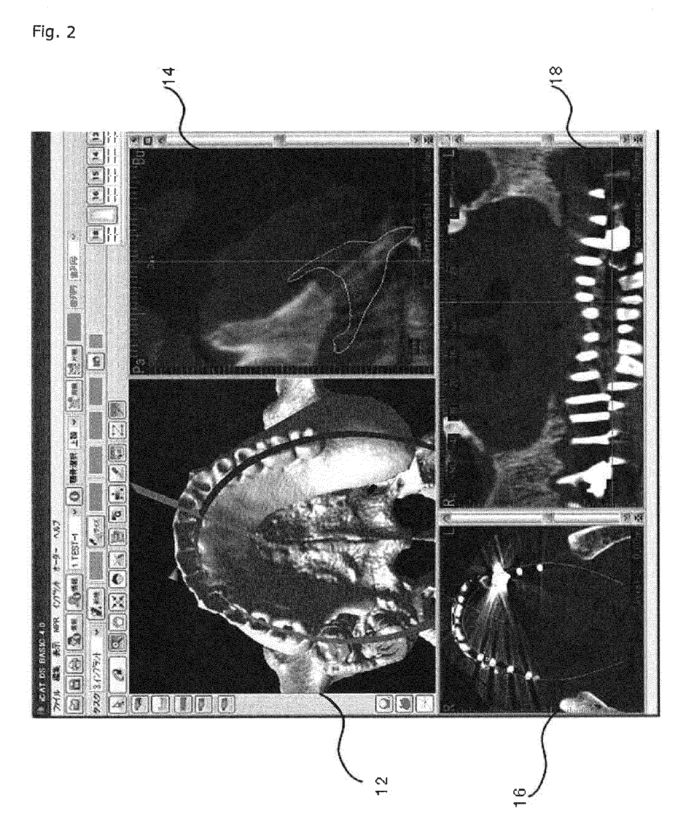

More specifically, a curved MPR 20 is set along the dental arch of maxilla in the three-dimensional window 12. FIG. 2 shows a state in which the maxilla is rotated in the three-dimensional window 12 and viewed from the inferior side. The cross-sectional image along the curved MPR 20 is displayed in the panoramic cross-section window 18 as a panoramic cross-sectional image. In FIG. 1, for the sake of easier view, the panoramic cross-sectional plane 20 is omitted in the three-dimensional window 12.

Going back to FIG. 1, once the panoramic cross-sectional plane 20 is set, a panorama-orthogonal cross-sectional plane 22 is set. This panorama-orthogonal cross-sectional plane 22 extends in the superior-inferior directions and perpendicularly intersects the panoramic cross-sectional plane 20. A two-dimensional image on the panorama-orthogonal cross-sectional plane 22 is displayed in the panorama-orthogonal cross-section window 14.

As described later, the panorama-orthogonal cross-section can tilt toward the panoramic cross-section. To do this, it is necessary to set the tilting center. First, a horizontal cross-sectional plane 24 appears in the three-dimensional window 12 at a certain height in the superior-inferior direction. This horizontal cross-sectional plane 24 is movable in the superior-inferior direction, and set at a desired height. The two-dimensional image on the horizontal cross-sectional plane 24 is displayed on the horizontal cross-section window 16.

The line of intersection formed by the horizontal cross-sectional plane 24 and the panorama-orthogonal cross-sectional plane 22 is the rotational axis around which the panorama-orthogonal cross-sectional plane 22 is tilted.

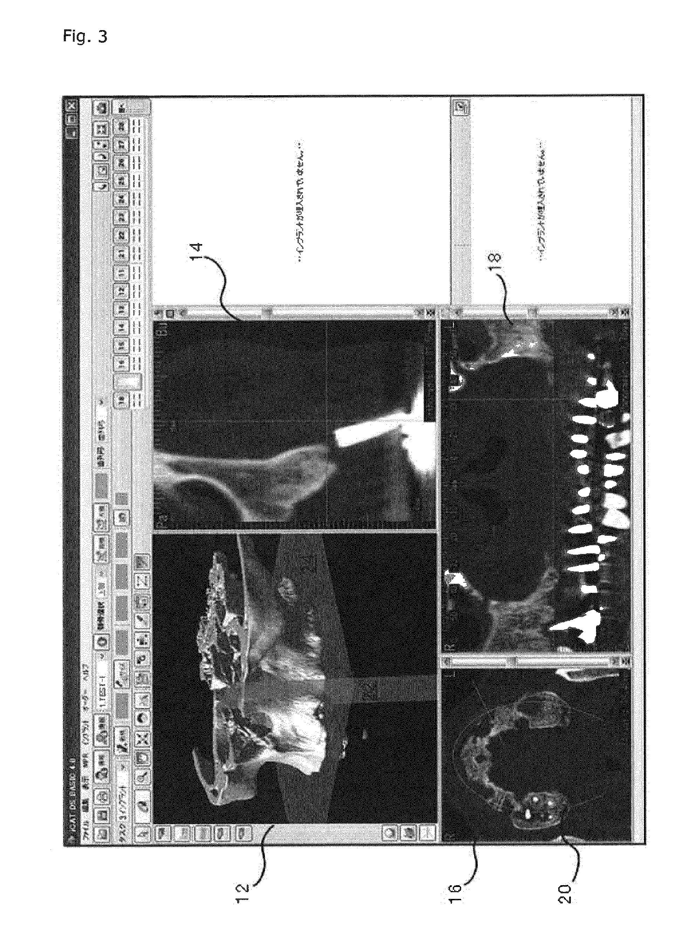

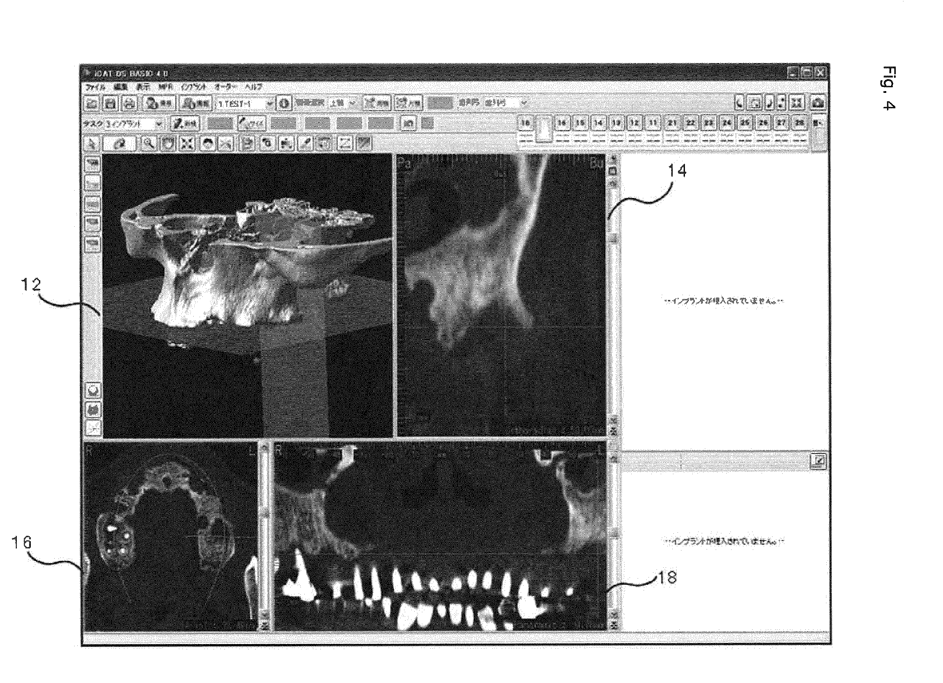

FIG. 3 shows a state where the panorama-orthogonal cross-sectional plane 22 has shifted along the panoramic cross-sectional plane 20. FIG. 4 shows a state where the panorama-orthogonal cross-sectional plane 22 has further shifted.

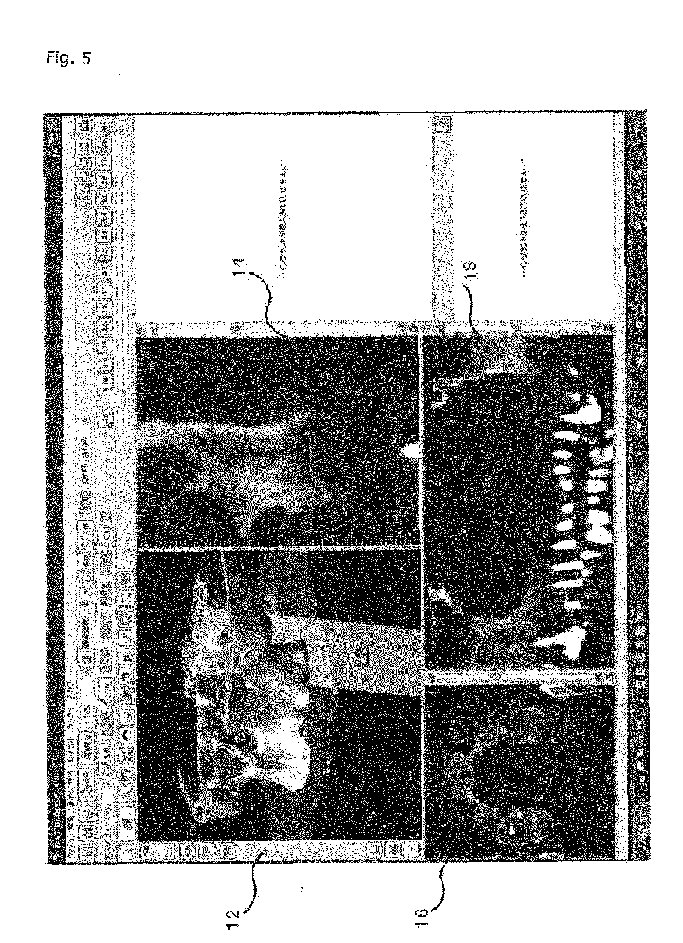

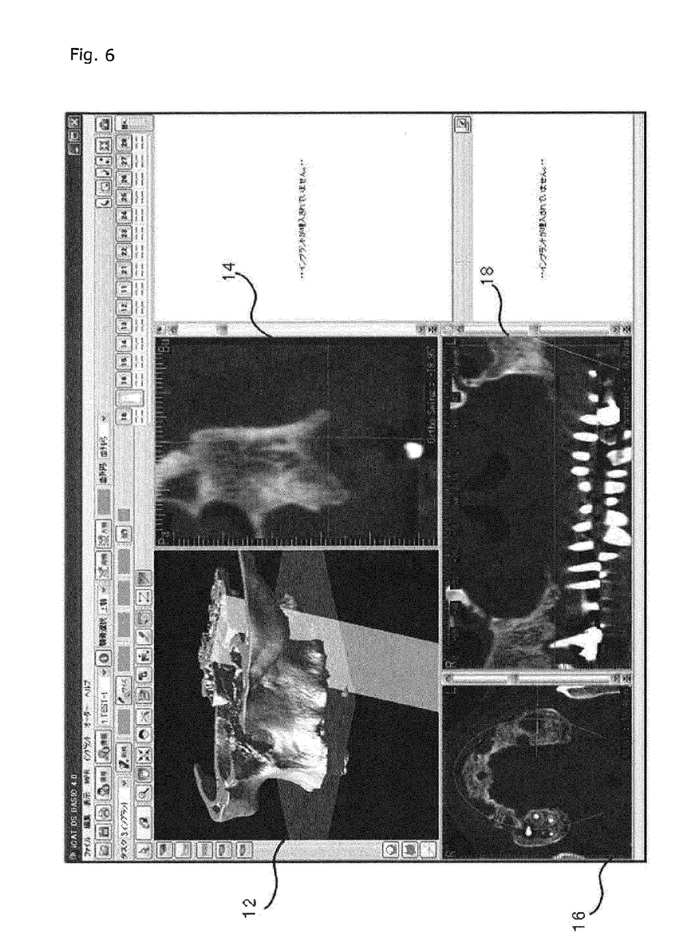

FIG. 5 shows a state where the panorama-orthogonal cross-sectional plane 22 has been tilted. FIG. 6 shows a state where the panorama-orthogonal cross-sectional plane 22 has been further tilted.

Although not shown in the picture, the tilted panorama-orthogonal cross-sectional plane 22 can further slide in the tilted direction of the panorama-orthogonal cross-sectional plane 22. In addition, the tilted panorama-orthogonal cross-sectional plane 22 can shift along the panoramic cross-section. Then, the image on the panorama-orthogonal cross-sectional plane can be displayed in the panorama-orthogonal cross-section window 14 as two-dimensional image information.

Furthermore, it is also possible to rotate the panorama-orthogonal cross-sectional plane 22 around the line of intersection formed by the panorama-orthogonal cross-sectional plane 22 and the panoramic cross-sectional plane 20.

According to the above embodiment, it is possible to view the tomographic images on the panorama-orthogonal cross-sectional plane 22, which is tilted, moved, slid and rotated. The body axes of the head and neck region displayed in the three-dimensional window 12 are assumed to correspond to the axes of the dimensions of the three-dimensional window 12. However, as described earlier, actually there are mismatches between the axes of the photographed body image and the axes of the three-dimensional window 12. According to the present two-dimensional image display device, it is possible to display the two-dimensional image, in which such axial mismatches are corrected, because the two-dimensional image is obtained from the absolute coordinates in the three-dimensional window 12. Particularly, when the picture is taken after placing or injecting a contrast material in the human body, the details of the body are enhanced by the contrast material as is well observed in the tomographic image. Therefore, the above-described two-dimensional image display device can be effectively utilized for implant treatments.

Although an embodiment of the present invention has been described above, the present invention is not limited to this. It is easily understood for a person ordinarily skilled in the art that there are improvements and modifications based on the specification and claims.

### Explanation of Numbers

- 12: Three-dimensional window
- 14: Panorama-orthogonal cross-section window
- 16: Horizontal cross-section window
- 18: Panoramic cross-section window
- 20: Panoramic cross-sectional plane
- 22: Panorama-orthogonal cross-sectional plane
- 24: Horizontal cross-sectional plane

## Claims

1. A two-dimensional image display device for displaying cross-sectional images around a jawbone based on three-dimensional image information generated from X-ray CT photographic data of a head and neck region, the cross-sectional images including an image on a panorama-orthogonal cross-sectional plane, which extends in a superior-inferior direction and is orthogonal to a panoramic cross-section, the device comprising:
a three-dimensional image display means for displaying a three-dimensional image of the head and neck region;
a horizontal cross-section display means for displaying a cross-sectional image on a horizontal cross-sectional plane, which is defined in the three-dimensional image display means;
a panoramic cross-section display means for displaying a panoramic cross-sectional image of the jawbone; and
a panorama-orthogonal cross-section display means for displaying the cross-sectional image on the panorama-orthogonal cross-sectional plane;
wherein the panorama-orthogonal cross-sectional plane is shiftable in a mesial-distal direction along the panoramic cross-section;
wherein the panorama-orthogonal cross-sectional plane is tiltable around a line of intersection formed by the panorama-orthogonal cross-sectional plane and the horizontal cross-sectional plane; and
wherein the panorama-orthogonal cross-section display means displays a cross-sectional image of the jawbone on the panorama-orthogonal cross-sectional plane, which is tilted from the panorama-orthogonal cross-section.

2. The two-dimensional image display device of claim 1, wherein the panorama-orthogonal cross-sectional plane tilted from the panorama-orthogonal cross-section is slidable in a direction of the tilt angle; and
wherein the panorama-orthogonal cross-section display means displays a cross-sectional image of the jawbone on the panorama-orthogonal cross-sectional plane, which is tilted from the panorama-orthogonal cross-section and slid in a direction of the tilt angle.

3. The two-dimensional image display device of claim 2, wherein the panorama-orthogonal cross-sectional plane tilted from the panorama-orthogonal cross-section is shiftable in a mesial-distal direction along the panoramic cross-section; and
wherein the panorama-orthogonal cross-section display means displays a cross-sectional image of the jawbone on the panorama-orthogonal cross-sectional plane, which is tilted from the panorama-orthogonal cross-section and shifted in the mesial-distal direction.

4. The two-dimensional image display device of claim 1 or 2, wherein the panorama-orthogonal cross-sectional plane is rotatable around a line of intersection formed by the panorama-orthogonal cross-sectional plane and the panoramic cross-section; and
wherein the panorama-orthogonal cross-section display means displays a cross-sectional image of the jawbone on the rotated panorama-orthogonal cross-sectional plane.

5. The two-dimensional image display device of claim 4, wherein the rotated panorama-orthogonal cross-sectional plane is slidable in a direction of the rotational angle; and
wherein the panorama-orthogonal cross-section display means displays a cross-sectional image of the jawbone on the panorama-orthogonal cross-sectional plane, which is rotated around the line of intersection and slid in a direction of the rotational angle.

6. The two-dimensional image display device of claim 4, wherein the rotated panorama-orthogonal cross-sectional plane is shiftable in a mesial-distal direction along the panoramic cross-section; and
wherein the panorama-orthogonal cross-section display means displays a cross-sectional image of the jawbone on the panorama-orthogonal cross-sectional plane, which is rotated around the line of intersection and shifted in the mesial-distal direction.
